# EUROPÄISCHE PATENTSCHRIFT

(11) **EP 1 654 989 B1**
(45) Veröffentlichungstag und Bekanntmachung des Hinweises auf die Patenterteilung: **17.12.2008**
(21) Anmeldenummer: 05023522.5
(22) Anmeldetag: 27.10.2005
(51) Int. Cl.: A61B 17/04, A61F 2/08

(54) **Ovaler Stift zum Fixieren eines unter Zuglast beanspruchten Implantates**
Oval pin for the fixation of an implant under load
Broche ovale pour la fixation d'un implant sous contraintes

(30) Priorität: 03.11.2004 DE 102004053464
(43) Veröffentlichungstag der Anmeldung: 10.05.2006
(73) Patentinhaber: Karl Storz GmbH & Co. KG, 78532 Tuttlingen (DE)
(72) Erfinder: Strobel, Michael J., Prof. Dr., 94315 Straubing (DE); Weiler, Andreas, Dr., 13505 Berlin (DE); Petersen, Wolf, Dr., 48149 Münster (DE); Berberich, Sascha, 78532 Tuttlingen (DE)
(74) Vertreter: Weller, Wolfgang

(56) Entgegenhaltungen:
- EP-A- 1 180 351
- EP-A- 1 297 799
- WO-A-00/30552
- WO-A-99/08625
- WO-A-20/04062459
- AU-B2- 767 853
- DE-U1- 29 910 202
- US-A1- 2001 053 934

## Beschreibung

Die Erfindung betrifft einen Stift zum Fixieren eines unter Zuglast beanspruchten Implantates, insbesondere eines Sehnenimplantates, mit einem stabförmigen Körper, auf dessen Querschnitt auf einer Seite die durch das Implantat ausgeübte Kraft einleitbar und auf der gegenüberliegenden Seite in eine Verankerungsstelle ableitbar ist.

Ein derartiger Stift ist aus der US 2001/053934 A1 bekannt.

Derartige Stifte dienen dazu, ein Implantat, das in einer Öffnung in einen Knochen eingesetzt ist, zu fixieren. Dazu wird zunächst das Implantat in die Öffnung, meist eine von der Außenseite her bewerkstelligte Bohrung, eingeschoben. Anschließend wird der Stift quer dazu eingetrieben, dabei die Bohrung und das darin eingeschobene Implantat durchquerend, wodurch dieses in der Lage fixiert ist.

Da der Stift quer zur Längserstreckung des Implantates verläuft, hat sich der Ausdruck "Cross Pin" etabliert.

Eine weitverbreitete Anwendung ist die Fixierung eines Implantates, das als Ersatz der Kreuzbänder im Knie dient.

Beim Ersatz des Kreuzbandes hat sich eine Operationstechnik entwickelt, bei der das Implantat, meist eine Sehne des Patienten, zu einer Schleife gelegt wird und der Cross Pin im Bereich der Schleife quer durchgetrieben wird.

Die Sehne umschlingt also den stabförmigen Körper in einer Querschnittsebene. Daraus resultiert, dass die von der Sehne ausgeübte Zuglast auf einer Querschnittsseite in den stabförmigen Körper eingeleitet wird und auf der gegenüberliegenden Seite in eine Verankerungsstelle ableitbar ist. Diese Verankerungsstelle stellt die Innenwand, geometrisch gesehen eine Mantellinie dieser Wand, einer Bohrung dar, in der der Cross Pin aufgenommen ist.

Üblicherweise haben solche Stifte einen kreisförmigen Querschnitt, so dass die kraftableitende Stelle längs einer Mantellinie des stabförmigen Körpers liegt, die der Schleife bzw. der Querschnittseite gegenüberliegt, um die die Schleife geführt ist.

Dies führt im Belastungsfall dazu, dass die durch das Sehnenimplantat eingeleitete Kraft gegenüberliegend an einer mehr oder weniger begrenzten Stelle in die Verankerungsstelle abgeleitet wird.

Es sind auch Operationstechniken bekannt, bei denen der Cross Pin direkt durch die Sehne bzw. zusammengenähten Sehnenabschnitte getrieben wird.

Es wurde durch eine Studie festgestellt, dass es bei dieser Geometrie zu einem Bruch des Cross Pins kommen kann.

Um dem abzuhelfen, wurde versucht, in Richtung der Krafteinwirkung unter dem Cross Pin unmittelbar benachbart noch einen zweiten gleichen Cross Pin mit ebenfalls kreisförmigem Querschnitt einzubringen.

Eine weitere Studie hat nun gezeigt, dass auch das Setzen eines zweiten benachbarten Cross Pins einen Bruch nicht ausschließen kann, ganz im Gegenteil, wenn der erste Cross Pin bei einer starken Zugbelastung bricht, so bricht im Anschluss daran auch gleich der zweite Cross Pin; es ist quasi eine Art Dominoeffekt vorhanden.

Aus der EP-A-1 297 799 ist ein Stift zum Fixieren eines unter Zuglast beanspruchten Sehnenimplantates bekannt. Der Stift weist einen stabförmigen Körper auf, auf dessen Querschnitt auf einer Seite die durch das Implantat ausgeübte Kraft einleitbar und auf der gegenüberliegenden Seite in eine Verankerungsstelle ableitbar ist. Der Querschnitt in Richtung der vom Sehnenimplantat eingeleiteten Kraft weist ein längeres Ausmaß als quer zur Längsachse auf.

In der WO 2004/062459 A sind Nahtanker beschrieben, die einen stabförmigen Körper aufweisen. Das Querschnittsprofil der stabförmigen Körper kann vielfältig ausgestaltet werden, z.B. rund, oval, rechteckig, dreieckig oder halbkreisförmig.

Es ist daher Aufgabe der vorliegenden Erfindung, hier Abhilfe zu schaffen und einen Stift bzw. Cross Pin der eingangs genannten Art zum Fixieren eines unter Zuglast beanspruchten Implantates zu schaffen, der hohen Zugbelastungen ohne Bruch widerstehen kann.

Erfindungsgemäß wird die Aufgabe dadurch gelöst, dass der Querschnitt in Richtung der vom Sehnenimplantat eingeleiteten Kraft ein längeres Ausmaß als quer zur Längsrichtung aufweist und dass der Querschnitt als Flachoval ausgebildet ist.

Es wurde festgestellt, dass durch ein Abweichen von einer runden Geometrie und eine Anordnung des Cross Pins derart, dass das längere Querschnittsmaß sich in Richtung der vom Sehnenimplantat eingeleiteten Kraft erstreckt, eine höhere Bruchsicherheit erzielt werden kann.

Ferner wurde als weiterer Vorteil festgestellt, dass die Sehne über einen relativ großen Umschlingungswinkel und über eine große Umschlingungsfläche das Implantat umgreift, wodurch eine flächenförmige Anpressung des Sehnenimplantates in der Bohrung resultiert, was das Einwachsen der Sehne begünstigt. Die Sehne wird außerdem stärker komprimiert als durch einen runden Pin, woraus eine bessere Fixierung resultiert.

Die Ausbildung als Flachoval ist nicht nur fertigungstechnisch einfach zu bewerkstelligen, sondern diese Geometrie entspricht etwa der Umhüllungskurve zweier nebeneinanderliegender Pins mit kreisförmigem Querschnitt. Das bedeutet, dass hier auch nicht mehr Raum zur Verfügung stehen muss als bei den eingangs erwähnten Operationstechniken mit zwei nebeneinanderliegenden Pins. Man erzielt aber eine wesentlich höhere Bruchfestigkeit.

Das bedeutet aber nicht, dass ein Pin mit einem ovalären Querschnitt nun zwingend die Größe einer Umschlingungsbahn zweier herkömmlicher Pins mit kreisförmigem Querschnitt aufweisen muss, es kann durchaus sein, dass die längere stehende Hochachse des Querschnittprofils dem Durchmesser eines herkömmlichen Pins entspricht.

Wesentlich ist, dass in Richtung der Krafteinwirkung ein längeres Ausmaß gegeben ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger Ausführungsbeispiele im Zusammenhang mit den beiliegenden Zeichnungen näher beschrieben und erläutert. Es zeigen:
- Fig. 1: einen Stift bzw. Cross Pin in Seitenansicht;
- Fig. 2: einen Schnitt längs der Linie II-II in Fig. 1 in einem etwas vergrößerten Maßstab, wobei ein Sehnenimplantat, das durch den Stift fixiert werden soll, angedeutet ist, und
- Fig. 3: stark schematisiert einen in einen Oberschenkelknochen eingesetzten Stift von Fig. 1 zur Transfixation eines unter Zuglast beanspruchten Implantates.

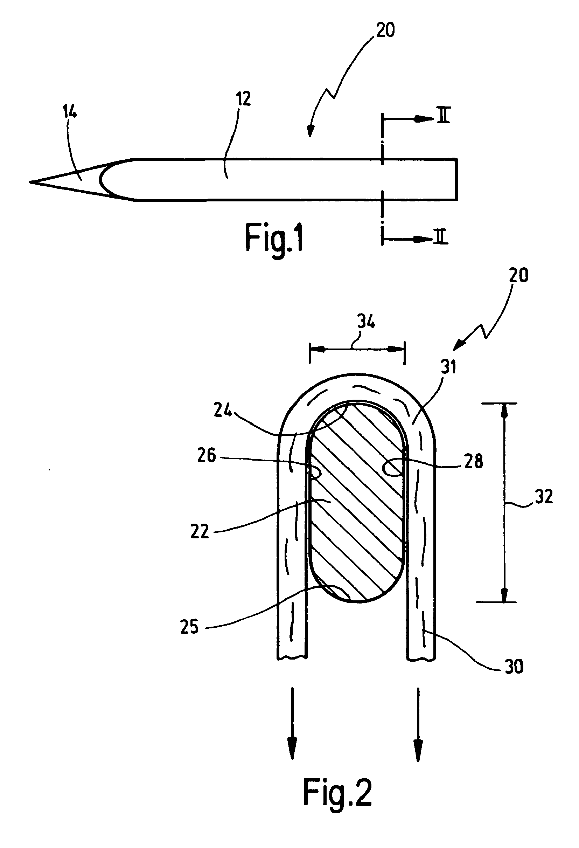

Ein in den Fig. 1 und 2 dargestellter Stift bzw. Cross Pin 20 weist einen stabförmigen Körper 12 auf, der an einer Seite in einer konisch zulaufenden Spitze 14 mündet.

In Fig. 2 ist der Stift 20 dargestellt, der einen Querschnitt aufweist, der als Flachoval 22 ausgebildet ist.

Das Flachoval 22 wird gegenüberliegend durch zwei Halbrunde 24 und 25 begrenzt, die über zwei parallel zueinander verlaufenden Seitenflanken 26 und 28 verbunden sind.

Es ist ferner dargestellt, wie ein Sehnenimplantat 30 um den Cross Pin 20 gelegt ist, und zwar in Form einer Schleife 31.

Der Cross Pin 20 ist dabei so ausgerichtet, dass dessen lange Achse 32 hochstehend ist, also in Richtung der Krafteinleitung durch das Sehnenimplantat. Die kurze Achse 34 verläuft quer dazu und auch quer zur Längsachse des Cross Pins 20.

Die Pins weisen beispielsweise eine Länge von ca. 40 mm und einen Durchmesser von etwa 4,5 mm auf.

Sie können aus resorbierbaren Materialien, aus Kunststoffmaterial oder auch aus metallischem Material, insbesondere Titan oder einer Titanlegierung, hergestellt werden.

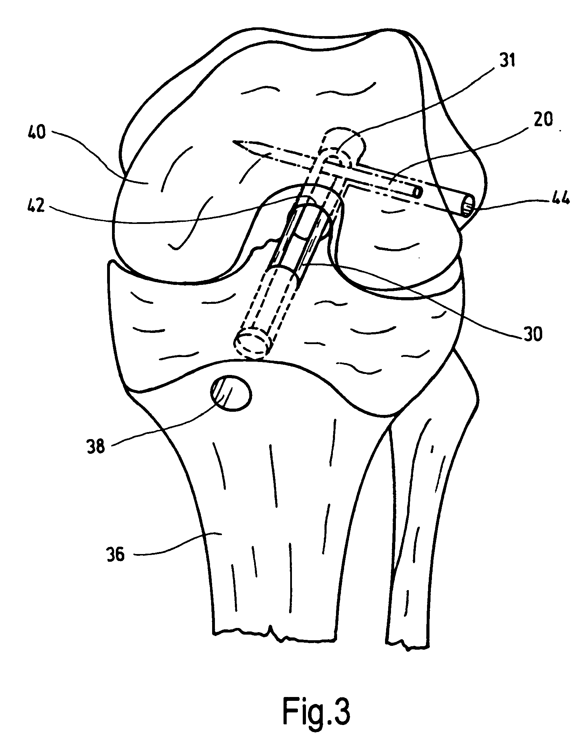

In Fig. 3 ist eine Situation dargestellt, bei der der Stift 20 bzw. Cross Pin zur Fixation eines Sehnenimplantates 30 dient, das als Ersatz eines Kreuzbandes im Knie eines Menschen dient.

Dazu wurde in der Tibia 36 eine durchgehende Bohrung 38 eingebracht, die sich im Femur 40 als Sacklochbohrung 42 fortsetzt.

Ferner ist im Femur 40 eine Querbohrung 44 eingebracht, um den Stift bzw. Cross Pin 20 eintreiben zu können, und zwar so, dass er durch die Schleife 32 des Sehnenimplantates 30 hindurch reicht.

Die Operationstechnik bzw. die notwendigen Hilfsgeräte zum Einbringen der Bohrungen 38, 42 sowie zum zielgerechten Einbringen der Querbohrung 44 bzw. zum Setzen und Befestigen des Implantates und des Cross Pins sind insbesondere in der US 2003/0065391 A1 und in der US 5,601,562 beschrieben, auf deren Inhalt ausdrücklich diesbezüglich Bezug genommen wird.

Soll ein Cross Pin mit flachovalem Querschnitt eingesetzt werden, wird zunächst mit einem Bohrer die Querbohrung 44 mit rundem Querschnitt bewerkstelligt, und anschließend wird ein Dilatator mit der etwa gleichen Kontur des Cross Pins 20 eingetrieben. Der Dilatator ist dabei etwas kleiner als der Cross Pin 20, so dass beim Einbringen des Cross Pins 20 ein Presssitz entsteht. Der Cross Pin wird jeweils so eingebracht, wie dies in Fig. 2 ersichtlich ist, also dass dessen längere Querschnittsachse 32 sich in Richtung der Krafteinwirkung oder Zugrichtung des Sehnenimplantates 30 erstreckt.

## Patentansprüche

1. Stift zum Fixieren eines unter Zuglast beanspruchten Implantates, insbesondere eines Sehnenimplantates (30), mit einem stabförmigen Körper (12), auf dessen Querschnitt auf einer Seite die durch das Implantat ausgeübte Kraft einleitbar ist, und auf der gegenüberliegenden Seite in eine Verankerungsstelle ableitbar ist, wobei der Querschnitt in Richtung der vom Sehnenimplantat (30) eingeleiteten Kraft ein längeres Ausmaß (32) als quer (34) zur Längsachse aufweist **dadurch gekennzeichnet, dass** der Querschnitt als Flachoval ausgebildet ist, wobei das Flachoval gegenüberliegend durch zwei Halbrunde (24 und 25) begrenzt wird, die über zwei parallel zueinander verlaufende Seitenflanken (26 und 28) verbunden sind.

## Claims

1. Pin for fixing an implant subjected to tensile load, in particular to a tendon implant (30), said pin having a rod-shaped body (12), the force exerted by the implant being able to be input onto the cross section of the body on one side, and being able to be output on the opposite side to an anchoring site, wherein the cross section has a longer dimension (32) in the direction of the force input from the tendon implant (30) than it has transverse (34) to the longitudinal axis, **characterized in that** the cross section is designed as a flat oval, wherein the flat oval is, at its opposite ends, delimited by two semi circles (24 and 25) which are connected via two mutually parallel side flanks (26 and 28).

## Revendications

1. Broche pour la fixation d'un implant soumis à une contrainte de traction, notamment d'un implant tendineux (30), avec un corps en forme de tige (12) sur la section duquel la force exercée par l'implant peut être introduite sur un côté et peut être dissipée dans un point d'ancrage sur le côté opposé, sachant que la section présente une plus grande dimension (32) dans la direction de la force introduite par l'implant tendineux (30) que transversalement (34) à l'axe longitudinal, **caractérisée en ce que** la section est réalisée sous la forme d'un ovale plat, sachant que l'ovale plat est délimité par deux demi-ronds (24 et 25) en opposition qui sont reliés par l'intermédiaire de deux flancs latéraux (26 et 28) s'étendant parallèlement entre eux.
